# EUROPEAN PATENT APPLICATION

(11) **EP 4 080 518 A1**
(43) Date of publication of application: **26.10.2022**
(21) Application number: 21386025.7
(22) Date of filing: 19.04.2021
(51) Int. Cl.: G16H 40/20, G06N 3/00, G06N 20/00, G16H 50/70

(54) **CLINICAL DECISION SUPPORT SYSTEM ON WEARABLE DEVICE**

(71) Applicant: VIDAVO S.A., 57001 Thessaloniki (GR); Fourlis, Alexios, 57500 Thessaloniki (GR); Psymanou, Markella, 55132 Thessaloniki (GR); Angelidis, Pantelis, 54646 Thessaloniki (GR)
(72) Inventor: Fourlis, Alexios, 57500 Thessaloniki (GR)

(57) **Abstract**

The invention concerns a wearable device equipped with biosensors able to provide support on physician's decisions by deploying an embedded Clinical Decision Support System (CDSS) exploiting edge computing. The CDSS monitors continuously vital signs, triages Covid-19 patients and reassigns priorities in Emergency Departments. Moreover, the invention realizes a probabilistic framework able to notify physicians about the probabilities of a patient a) to need hospitalization and b) to be admitted in the Intensive Care Unit (ICU). Additionally, the invention is interconnected with supportive software components in order to notify physicians about any health deterioration.

## Description

The invention concerns a wearable device equipped with biosensors and multiple wireless data transmission capabilities able to provide support on physician's decisions by deploying an embedded Clinical Decision Support System (CDSS) exploiting edge computing technologies and techniques. The wearable device is composed by a) the main processor unit (dual-core processor and a cryptographic accelerator), b) 0.96 IPS screen c) 9-axis Inertial Measurement Unit (IMU) and a two-channel wavelength Photoplethysmogram (PPG) optical sensor and d) Integrated circuit in order to support 2.4 GHz Wi-Fi, Bluetooth Low Energy protocols and Bluetooth 5.0.

Exploiting edge computing and advanced digital signal processing techniques on PPG and IMU signals, the wearable device can continuously extract the following vital signs: a) Heart Rate (HR), b) Heart Rate Variability (HRV), c) Respiratory Rate (RR), d) Blood Oxygen Saturation (SpO2), e) Body Temperature (BT), f) Blood Pressure (BP) and g) Blood Glucose (BG). The invention is intended to be used in Emergency Departments (ED) in order to automatically triage and prioritize patients based on their medical status severity.

Providing proper combinations of the aforementioned vital signs, healthcare personnel are able to make an estimation of the severity and have a clinical overview for a variety of diseases and medical conditions in ED inflows. For example, the combination of BT, SpO2 and RR are valuable for monitoring SARS-COV-2 (Covid-19), while combining BP, HR and RR valuable conclusions can be extracted for Cardiovascular Diseases.

Triage and prioritization of patients based on their medical status severity is the most crucial procedure that takes place when patients arrive at EDs. Medical experts are using triage algorithms to set the medically correct order of serving incoming patients. Commonly triage algorithms are expecting as input vital signs, current symptoms and short medical history (pre-triage procedure) in order to stratify patients to severity classes. While the severity of most classes is obvious (e.g., hand fracture is low while heart attack is extremely high), there is a severity class for which is not possible to extract safe conclusions from the pre-triage procedure, therefore several exams (blood test, x rays, CT-scanner etc.) must take place in order to give a better overview to physicians. In many cases, these exams are extremely time consuming and continuous monitoring of these patients is mandatory in order to avoid fatal incidents, while the application of CDSS is a significant tool to minimize physicians' errors and provide medical attention and care in time.

The significant novelty of the invention is the use of Artificial Intelligence (AI) techniques on the wearable. Exploiting edge computing, the invention is able to deploy the CDSS on the wearable avoiding to transmit raw data over a radio channel to expensive, power hungry and vulnerable cloud infrastructures, ensuring data privacy, security, energy efficiency and minimizing latency. It is worth mentioning that the Neural Network (NN) is not just extracting vital signs but identifies health dysfunctions depending on other parameters too, like age, height, weight, symptoms and short medical history, realizing the CDSS. These data are available from the supportive software components where users are registered, aiming to add intelligence and have more accurate results in real world applications.

The supportive software components are mobile and web applications and backend services, able to execute basic functions in order to register and manage patients, as well as to receive notifications from the CDSS and provide interactive visualization dashboards. It is not mandatory for the wearable device to be aware of all of the aforementioned variables but if any of them is available is taken into account.

Furthermore, the CDSS realizes a probabilistic framework able to notify physicians about the probabilities of a patient a) to need hospitalization and b) to be admitted in the Intensive Care Unit (ICU). To this end, the CDSS extends the invention's value regarding medical data and provides projection insights on Hospital's resources. This framework is dynamic and during patients' journey inside the EDs are recalculated on real-time.

The fact that the invention exploits edge computing technologies establishes a significant advantage against current state-of-the-art solutions. It realizes a more feasible and sustainable business case, where users are not frustrated by solutions that do not work or miss important events when internet connection is unavailable. For example, in a Hospital environment, where sending data out of the physical area of the hospital is forbidden, the invention is able to provide CDSS results without having internet connection at any stage of data processing.

Furthermore, the device offers a patient's indoor positioning system exploiting Wi-Fi. The wearable device in case of an emergency notifies the healthcare personnel letting them know at the same time about the area where the patient is.

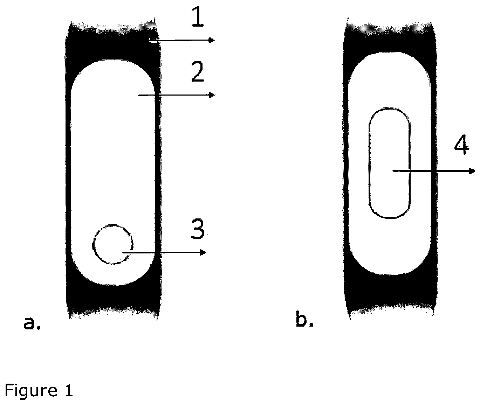

Figure 1a. presents the upper side of the wearable device, where a silicone strap (1) is used to apply steady pressure to patients' wrists, a procedure that is necessary in order for the PPG analysis to be credible. The upper side of the wearable device is almost covered by the 0.96 IPS screen (2), while it is equipped with a capacitance button (3) in order for the users to interact with the device. It is worth mentioning that the screen is used only for over the air firmware update because for the invention there is no value to present the vital signs and the CDSS results or any other information to patients. The PPG sensor is placed on the down side of the wearable device (4), as figure 1b. presents, where it is equipped with an anti-aliasing glass in order to have maximum usage of reflective radiation.

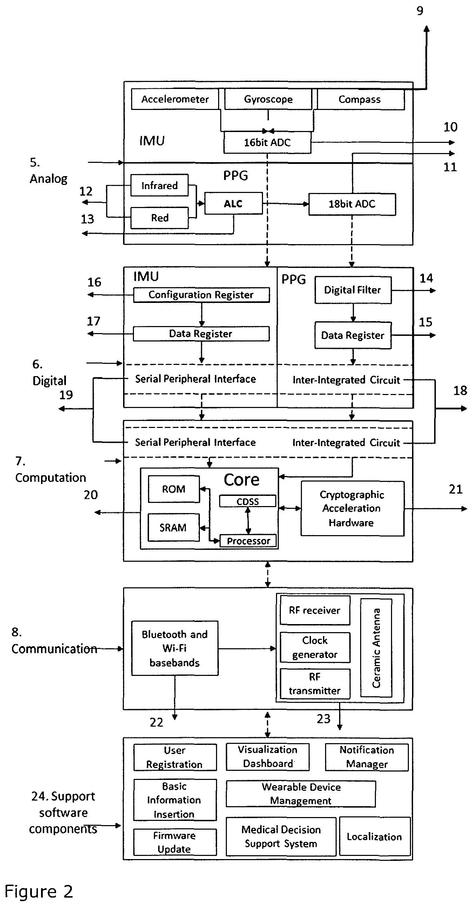

Figure 2 presents the functional block diagram of the wearable device and more specifically the raw IMU and PPG signals processing in order to convert them into digital information able to perform digital signal processing (DSP), feed the NN and extract the aforementioned results. The signals processing is consecutively implemented by the analogue (5), the digital (6) and the computation (7) parts. More precisely, the IMU (9) and the PPG (11) sensors are extracting 11 raw analog signals; 9-axis and raw red and infrared radiation. Therefore, two separate analog-to-digital converters (ADC) are used in order for the signals to be processable from the digital block; 16bit for the IMU (10) and 18bit for the PPG (11). Raw red and infrared signals are applied to the Ambient Light Cancellation block in order to increase the effective dynamic radiation range avoiding the environmental light (13).

Next, a hardware digital filter is applied to the PPG signal in order to cancel digital noise (14) and to be stored to Data Register (15). Data Register is a 192 bytes FIFO structure able to store 32 samples of red and infrared data. Then using an Inte-Integrated Circuit (I2C) embedded interface (18), FIFO data are available to wearable device core (20). Regarding the IMU, after the signals are applied to 16bit ADC (10), they are adjusted according to the configuration register (16), where their resolution and scale are defined. The Data Register (17) is a read-only 512-byte FIFO structure containing IMU data accessible via the Serial Peripheral Interface (19) from the wearable device core (20). At this point, the Core of the wearable device has access to sensors' data in order to perform DSP, execute the NN in order to perform the predictive analytics. Whenever the results are extracted, they are forwarded to Cryptographic Acceleration Hardware block (21) in order to be cryptographed by AES algorithm with 256-bit key.

The cryptographed results are forwarded to the Communication block (8), where the baseband protocols for Wi-Fi and Bluetooth are carried out, as well as other low-level connection functions, such as modulation, demodulation, packet processing, bit stream processing, frequency hopping, etc. (22). Exploiting the radio frequency integrated circuit (23) data are transmitted to supported software components (24) and received by them according to the use case that the invention is used.

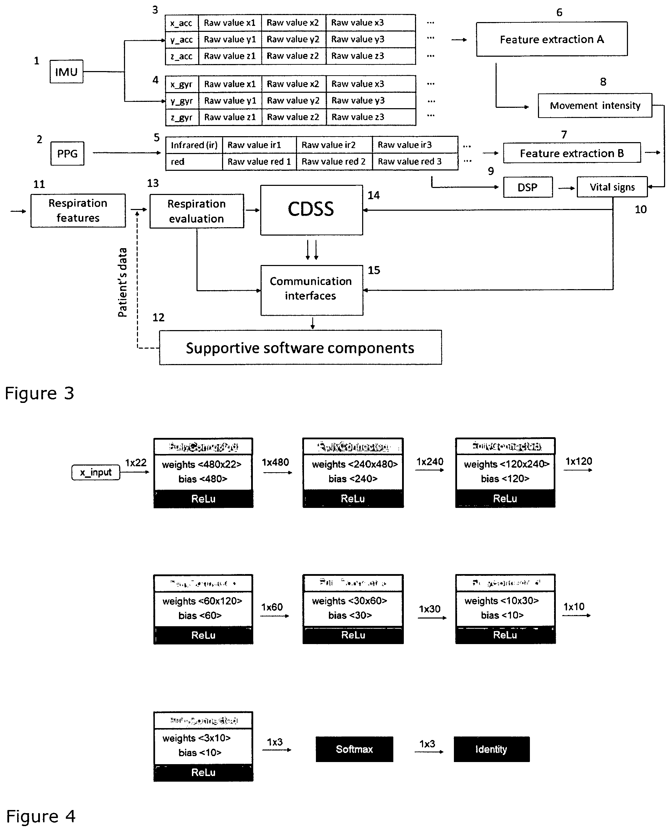

Figure 3 gives a better understanding of what the CDSS is and how it is executed locally on the wearable device. The CDSS is a NN closed-source software component expecting a set of variables in order to extract the aforementioned results and probabilities. IMU (1) and PPG (2) sensors are continuously inserting a specific number of raw acceleration (3), gyroscope (4), infrared and red radiation to Feature extraction A (6) and B (7), respectively.

Feature extraction A consists of power spectral density and root mean square calculations in order to extract features regarding movement intensity (8). Taking in account the extracted movement intensity, a set of DSP techniques (9) are applied on raw PPG in order to calculate the vital signs (10). Simultaneously, Fast Fourier Transform (7) is applied on the same raw PPG signal in order to extract respiration features (11).

At this point, the wearable device asks from the supportive software components (12) patient's data in order to evaluate respiration (13). The extracted vital signs, patient's data and the evaluation of respiration are inserted to the CDSS (14) which calculates the probabilities. The CDSS results, the vital signs and the respiration evaluation are available to the supportive software components (12), and to extend to physicians, through the communication interfaces (15) of the wearable device. The above procedure, except from the asking data procedure, is repeated until the patient's monitoring stops.

The CDSS is a pre-trained machine learning model compressed in a header file optimized for memory limited embedded systems. The model architecture consists of consecutive regular densely-connected layers in pyramid format. Figure 4 presents the algorithm executed by the CDSS on the processor. The CDSS expects as an input the exact 22 features that were used for training. These 22 features are described in figure 3. The first layer of the model consists of 480 nodes. CDSS stores the 22-input features into a 22 element array. Each element is copied to each of the nodes in the first layer. Then, each node multiplies each input feature by a weight and adds the constant bias in order to sum them altogether. The sum is passed through a nonlinear function, the rectified linear unit (ReLu), to give each node its decision-making ability.

Specifically, it acts as the activation function being responsible for transforming the summed weighted input from the node into the activation of the next node or output for that input. This output is copied to every node in the second layer. The process repeats again, but with a different set of nodes; 240 for the second layer. This procedure is repeated until the last node at the seventh layer.

Finally, there is one node for each possible class in the output layer, e.g., 3 possible outputs, hence 3 possible classes. The same math is performed with the weights and bias terms, but instead of the ReLu, the summation of the nodes is sent to the Softmax function in order to convert all of these outputs into probabilities for each class; 1) Probability the patient to be hospitalized and b) Probability the patient to be admitted in the Intensive Care Unit (ICU) and 3) Input features indicate an unknown anomaly and the CDSS is not confident enough.

The algorithms described and presented on figures 3 and 4 are taking 138 milliseconds for the processor to execute them and receive results. The pre-trained machine learning model requires 658 kB of ROM, maximum of 5.8KB of RAM, has accuracy of 95.1% and an average of 0.95 F1 score.

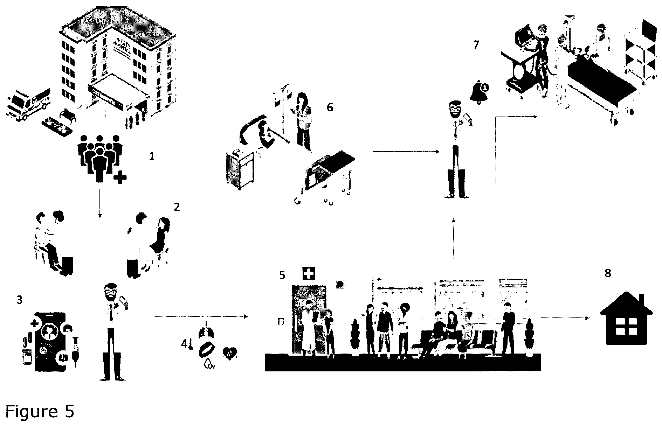

Figure 5 presents a typical use case of Covid-19 suspicious cases arriving to Emergency Departments (ED) (1) and how the invention is applied. All patients that suffer from Covid-19 related symptoms undergo a pre-triage procedure (2) for identification of suspected or confirmed COVID-19 cases according to the World Health Organisation criteria. Those who meet the criteria are shown to the Covid-19 ED area, where triage and prioritization algorithms and protocols are performed by specialized physicians.
Based on the results, patients' category that the applied protocol refers as the intermediate severity and medical attention is required, are registered to the system through the mobile application (3). Questions concern demographics, vital signs, past medical history and main presenting symptoms are composing patients' profiles. Using the supportive software components, these profiles are shared with the rest of the system and are linked with one wearable device.

At this point, the physician installs the wearable device and it starts continuous monitoring of the patient's vital signs. For the Covid-19 example, the vital signs that it is worth monitoring are HR, RR, SpO2 and BT. The wearable device is aware of the patient's profile that it is installed on, enabling the detection of potential patient's health deterioration that demands immediate medical attention by exploiting the CDSS (4).

Regardless of the patient journey in the EDs, physicians are able to monitor patients' vital signs and their location and receive notifications from the CDSS (5). Based on CDSS notifications and the correlation with the clinical overview, physicians are able to decide whenever patients should be hospitalized (6), admitted to the ICU (7) or give them medical instructions in order to recover at home (8). Related work was published in 2020 IEEE Globecom¹.
¹ Barbara Fyntanidou, Maria Zouka, Aikaterini Apostolopoulou, Panagiotis D. Bamidis, Antonis Billis, Konstantinos Mitsopoulos, Pantelis Angelidis, Alexis Fourlis "IoT-based smart triage of Covid-19 suspicious cases in the Emergency Department", IEEE GLOBECOM 2020 Special Workshop on Communications and Networking Technologies for Responding to COVID-19, Taipei, Taiwan, December 2020, [
.

## Claims

1. An algorithm that dynamically triages, assigns priorities and supports medical decisions regarding Covid-19 stratification cases in EDs independently from the triage algorithms that physicians may use in 138 milliseconds or less, requiring 658 kB of ROM and a maximum of 5.8KB of RAM.

2. Wearable medical device that dynamically triages, assigns priorities and supports medical decisions regarding Covid-19 stratification cases in EDs ensuring data privacy, security, energy efficiency and minimizing latency, while achieving an accuracy of 95.1% and an average of 0.95 F1 score.

3. A method (CDSS) extracting prediction probabilities of a patient a) to be hospitalized and b) to be admitted in the Intensive Care Unit (ICU).
